(19) Europäisches Patentamt
European Patent Office
Office européen des brevets

(11) **EP 3 795 074 B1**

(12) **EUROPEAN PATENT SPECIFICATION**

(45) Date of publication and mention
of the grant of the patent:
**27.04.2022 Bulletin 2022/17**

(21) Application number: **19891833.6**

(22) Date of filing: **08.10.2019**

(51) International Patent Classification (IPC):
*A61B 5/358* (2021.01)   *G16H 40/63* (2018.01)
*G16H 50/20* (2018.01)   *A61B 5/00* (2006.01)
*A61B 5/0245* (2006.01)

(52) Cooperative Patent Classification (CPC):
**A61B 5/358; A61B 5/7225; A61B 5/725;**
**G16H 40/63; G16H 50/20;** A61B 5/0245;
A61B 5/353; A61B 5/355; A61B 5/36; A61B 5/726

(86) International application number:
**PCT/CN2019/109884**

(87) International publication number:
**WO 2020/114068 (11.06.2020 Gazette 2020/24)**

(54) **METHOD AND DEVICE FOR AUTOMATICALLY DETERMINING ST SEGMENT OF ELECTROCARDIOGRAM SIGNAL BASED ON ARTIFICIAL INTELLIGENCE TECHNOLOGY**

VERFAHREN UND VORRICHTUNG ZUR AUTOMATISCHEN BESTIMMUNG DER ST-STRECKE VON ELEKTROKARDIOGRAM SIGNALEN AUF DER GRUNDLAGE EINER TECHNOLOGIE DER KÜNSTLICHEN INTELLIGENZ

PROCÉDÉ ET APPAREIL DE DÉTERMINATION AUTOMATIQUE DE SECTION ST DE SIGNAL D'ÉLECTROCARDIOGRAPHIE SUR LA BASE D'UNE TECHNOLOGIE D'INTELLIGENCE ARTIFICIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2018 CN 201811497524**

(43) Date of publication of application:
**24.03.2021 Bulletin 2021/12**

(73) Proprietor: **Shanghai Sid Medical Co., Ltd Shanghai 20000 (CN)**

(72) Inventors:
• **ZHU, Junjiang**
**Shanghai 20000 (CN)**
• **ZHANG, Detao**
**Shanghai 20000 (CN)**
• **WU, Shangshi**
**Shanghai 20000 (CN)**
• **WANG, Zhaoyang**
**Shanghai 20000 (CN)**
• **CHEN, Guangyi**
**Shanghai 20000 (CN)**

(74) Representative: **Petculescu, Ana-Maria Bayramoglu Law Offices LLC Türkiye Irtibat Ofisi, Mira Office, Kanuni Sultan Süleyman Boulevard, 5387. street Beytepe, floor 12, no:50 06800 Cankaya, Ankara (TR)**

(56) References cited:
CN-A- 101 028 186   CN-A- 104 173 043
CN-A- 107 951 485   CN-A- 108 618 777
CN-A- 109 620 214   US-A- 4 930 075
US-A1- 2011 040 200   US-A1- 2014 088 449
US-A1- 2015 272 464

• **KUMAR AMIT ET AL: "Ischemia detection using Isoelectric Energy Function", COMPUTERS IN BIOLOGY AND MEDICINE, NEW YORK, NY, US, vol. 68, 18 November 2015 (2015-11-18), pages 76-83, XP029383616, ISSN: 0010-4825, DOI: 10.1016/J.COMPBIOMED.2015.11.002**

EP 3 795 074 B1

**Description**

**TECHNICAL FIELD**

The present invention relates to the technical field of data processing, and in particular, to an artificial intelligence (AI)-based method and device for automatically determining an ST segment of an electrocardiogram signal.

**BACKGROUND**

An electrocardiogram is the comprehensive reflection of bioelectric signals on the body surface during the process of heart activity. Each cardiac cycle is usually composed of a series of P waves, QRS complex and T waves, wherein the QRS complex includes a Q wave, an R wave, and an S wave. The time interval between the start and the offset of each characteristic sub-waveband is significant, reflecting the physiological processes of the heart and autonomic nervous system. A segment from the end point of the QRS complex to the start point of the T wave is called the ST segment.

The ST segment represents the electric potential change in the repolarization process after the completion of ventricular depolarization. The normal ST segment should be on the equipotential line (baseline), horizontal or slightly inclined, and smoothly transition to the T waves gradually, and can have a slight upward or downward offset. Besides the ST segment, the other two typical equipotential lines are the TP segment and the PR segment. Compared with those, the abnormal electrocardiogram is beyond the normal deviation range when a certain part of the myocardium shows ischemia or necrosis. In this condition, there is still a potential difference after the completion of ventricular depolarization, and in this case, the ST segment shows deviation on the electrocardiogram, and the typical morphology is elevation and depression. The changes of the ST segment are important indexes for determining myocardial ischemia as well as myocardial infarction and acute pericarditis. Therefore, the accurate detection and determination of the morphological changes of the ST segment has important clinical significance.

At present, methods such as the time window detection method and the wavelet filtering method are used to extract and identify an ST segment. These methods have the advantages of simple calculation, easy implementation, and fast running speed. However, the morphology of ST segment varies widely, and these methods rely on the accuracy of identification of the feature point and the accuracy of extraction of the baseline. In instances when there is significant interference with the electrocardiogram signal or there are drift effects, however, the accuracy in identification and determination of the ST segment is greatly reduced.

US2011/040200 A1 relates to a system for analyzing an ECG signal comprising an interface that receives an ECG waveform associated with heart beat cycle of a patient. The system includes signal processor that determines a first isoelectric portion lying between a T-wave of a first heart beat cycle and a P-wave of a successive heart beat cycle, and a second isoelectric portion lying between a P-wave and a QRS complex of the first heart beat cycle. The signal processor determines a stability measure for each of said first and second portions and adaptively selects the first or the second portion as a baseline for the first heart beat cycle based on the stability measures. The signal processor determines a point of reference on an ST segment associated with the first heart beat cycle and evaluates a deviation of the point of reference on the ST segment from the selected baseline.

**SUMMARY**

In order to overcome the shortcomings of the prior art, an objective of the present invention is to provide an AI-based method and device for automatically determining an ST segment of an electrocardiogram signal. The present invention aims to solve the problem of the prior art that the accuracy in identification and determination of the ST segment is greatly reduced in instances when there is significant interference with the electrocardiogram signal or there are drift effects.

In order to achieve the above objective, the present invention adopts the following technical solutions.

An AI-based method for automatically determining an ST segment of an electrocardiogram signal, includes:

step S1: with respect to a filtered electrocardiogram signal s(t), under a premise that a position of an R wave peak is known, finding minimum values before and behind the R wave peak by partial enlargement to accurately locate a Q wave peak and an S wave peak;
step S2: performing wavelet transformation on an accurate position of a QRS complex obtained in step S1, and accurately locating a P wave peak and a T wave peak by the partial enlargement and a triangular area method;
step S3: with respect to the P wave peak and the T wave peak obtained in step S2, separately finding the start point and the end point of the P wave and the start point and the end point of the T wave in a search window by using the triangular area method;
step S4: with respect to the start point of the P wave obtained in step S3, pushing forward the start point of the P wave by a preset time to obtain the start point of a baseline TP segment, and locating the start point of an R wave according to the R wave peak by using the triangular area method;

step S5: with respect to the end point of the T wave obtained in step S3 and the known R wave peak, locating the start point of an ST segment by using an R+X method, and locating the end point of the ST segment by using the triangular area method, wherein the start point of the ST segment is marked as a point J, and the end point of the ST segment is marked as a point K;

step S6: performing average filtering on an electrocardiogram signal s(t) to obtain an average value sequence, defaulting the average value sequence as a baseline sequence $X_s$, and subtracting the baseline sequence $X_s$ from an original electrocardiogram signal s(t) to obtain a new electrocardiogram signal x(t);

step S7: extracting a baseline TP segment from the start point of the TP segment and the start point of the P wave located from step S1 to step S5, extracting a baseline PR segment from the located end point of the P wave and the located start point of the R wave, extracting a QQ connection line from two located adjacent Q wave peaks, and intercepting an electrocardiogram signal between the point J and the point K as the ST segment;

step S8: calculating a slope of each of the baseline TP segment, the baseline PR segment, and the QQ connection line obtained in step S7, calculating a heart rate of the new electrocardiogram signal x(t), and comprehensively determining a standard baseline for determining changes of the ST segment, a method for comprehensively determining the standard baseline includes:

> calculating the heart rate of the electrocardiogram signal x(t);
> when the heart rate is greater than 120, considering the heart rate to be excessively fast, and selecting the QQ connection line as the standard baseline; and
> when the heart rate is not greater than 120, calculating the slope KTP of the TP segment and the slope KPR of the PR segment; when KTP<KPR, selecting the TP segment as the standard baseline, otherwise, selecting the PR segment as the standard baseline.; and

step S9: with respect to the ST segment obtained in step S7 and the standard baseline obtained in step S8, calculating the difference between the average height of the ST segment and the average height of the baseline, and performing threshold determination according to characteristics of the electrocardiogram signal of different leads to determine whether the ST segment is elevated, depressed or normal.

Based on the above embodiment, preferably, in step S6, a method of subtracting the baseline sequence based on the average filtering adopts a moving average filtering function.

Based on the above embodiment, preferably, the moving average filtering function is expressed as follows:

$$X_s(i) = \frac{1}{2N+1}(s(i+N) + s(i+N-1) + ... + s(i-N))$$

wherein, $X_s(i)$ is an $i^{th}$ sampling obtained after smoothing an original electrocardiogram signal s(t); s(i) is an $i^{th}$ sampling point of the original electrocardiogram signal; N is the number of adjacent sampling points and is a positive integer, and N<0.05×sampling frequency.

Data is smoothed by replacing each sampling point with the average of N adjacent sampling points to obtain the baseline sequence $X_s$.

Based on the above embodiment, preferably, N=5.

An AI-based device for automatically determining an ST segment of an electrocardiogram signal, includes:

> a first module, wherein, with respect to a filtered electrocardiogram signal s(t), under a premise that a position of an R wave peak is known, the first module is configured to find minimum values before and behind the R wave peak by partial enlargement to accurately locate a Q wave peak and an S wave peak;
> a second module, wherein the second module is configured to perform wavelet transformation on an accurate position of a QRS complex obtained in the first module, and accurately locate a P wave peak and a T wave peak by the partial enlargement and a triangular area method;
> a third module, wherein, with respect to the P wave peak and the T wave peak obtained in the second module, the third module is configured to separately find the start point and the end point of the P wave and the start point and the end point of the T wave in a search window by using the triangular area method;
> a fourth module, wherein, with respect to the start point of the P wave obtained in the third module, the fourth module is configured to push forward the start point of the P wave by a preset time to obtain the start point of a baseline TP segment, and locate the start point of an R wave according to the R wave peak by using the triangular area method;

a fifth module, wherein, with respect to the end point of the T wave obtained in the third module and the known R wave peak, the fifth module is configured to locate the start point of an ST segment by using an R+X method, and locate the end point of the ST segment by using the triangular area method, wherein the start point of the ST segment is marked as a point J, and the end point of the ST segment is marked as a point K;

a sixth module, wherein the sixth module is configured to perform average filtering on an electrocardiogram signal s(t) to obtain an average value sequence, default the average value sequence as a baseline sequence $X_s$, and subtract the baseline sequence $X_s$ from an original electrocardiogram signal s(t) to obtain a new electrocardiogram signal x(t);

a seventh module, wherein the seventh module is configured to extract a baseline TP segment from the start point of the TP segment and the start point of the P wave located from the first module to the fifth module, extract a baseline PR segment from the located end point of the P wave and the located start point of the R wave, extract a QQ connection line from two located adjacent Q wave peaks, and intercept an electrocardiogram signal between the point J and the point K as the ST segment;

an eighth module, wherein the eighth module is configured to calculate a slope of each of the baseline TP segment, the baseline PR segment, and the QQ connection line obtained in the seventh module, calculate a heart rate of the new electrocardiogram signal x(t), and comprehensively determine a standard baseline for determining changes of the ST segment, wherein a method for comprehensively determining the standard baseline includes:

calculating the heart rate of the electrocardiogram signal x(t);

when the heart rate is greater than 120, considering the heart rate to be excessively fast, and selecting the QQ connection line as the standard baseline; and

when the heart rate is not greater than 120, calculating the slope $K_{TP}$ of the TP segment and the slope $K_{PR}$ of the PR segment; when $K_{TP}<K_{PR}$, selecting the TP segment as the standard baseline, otherwise, selecting the PR segment as the standard baseline; and

a ninth module, wherein, with respect to the ST segment obtained in the seventh module and the standard baseline obtained in the eighth module, the ninth module is configured calculate the difference between the average height of the ST segment and the average height of the baseline, and perform threshold determination according to characteristics of the electrocardiogram signal of different leads to determine whether the ST segment is elevated, depressed or normal.

Based on the above embodiment, preferably, in the sixth module, a method of subtracting the baseline sequence based on the average filtering adopts a moving average filtering function.

Based on the above embodiment, preferably, the moving average filtering function is expressed as follows:

$$X_s(i) = \frac{1}{2N+1}(s(i+N) + s(i+N-1) + \dots + s(i-N))$$

wherein, $X_s(i)$ is an $i$th sampling obtained after smoothing an original electrocardiogram signal s(t); s(i) is an $i$th sampling point of the original electrocardiogram signal; N is the number of adjacent sampling points and is a positive integer, and N<0.05 × sampling frequency.

Data is smoothed by replacing each sampling point with the average of N adjacent sampling points to obtain the baseline sequence $X_s$.

Based on the above embodiment, preferably, N=5.

Compared with the prior art, the present invention has the following advantages.

The present invention provides an AI-based method and device for automatically determining an ST segment of an electrocardiogram signal. With respect to the filtered human electrocardiogram signal, firstly, the key feature points of the electrocardiogram signal are separately extracted by combining the wavelet filtering and the triangular area method, including: locating the S wave and the T wave, and accurately identifying the start point and the slope characteristic of the ST segment. Secondly, a method for removing the baseline based on the average filtering is provided to subtract the extracted baseline sequence to obtain a new electrocardiogram signal. With respect to the new electrocardiogram signal, the ST segment and each baseline segment are extracted, and the heart rate and the slope of each baseline segment are calculated, and a standard baseline is comprehensively determined. Finally, the abnormal changes of the ST segment are identified based on the standard baseline to obtain the qualitative and quantitative determination result of the ST segment whether it is elevated, depressed, or normal.

According to the present invention, the feature points are first accurately located based on the original signal, and the ST segment and the baseline are extracted after removing the baseline sequence. Moreover, the standard baseline is comprehensively determined, which takes the inaccurate error during the extraction of the baseline caused by the baseline drift as well as other factors such as the heart rate which influences the baseline selection into consideration. In this way, the present invention solves the problems of the baseline error caused by the baseline drift, the inaccurate selection of the standard baseline, and the large difference in the number of abnormal heart beats, thereby indirectly improving the accuracy of the determination of the abnormal changes of the ST segment. The present invention has a simple calculation, can be easily implemented, and thus provides a new way for rapid and effective identification of the electrocardiogram signals.

**BRIEF DESCRIPTION OF THE DRAWINGS**

The present invention will be further described in the following drawings and embodiments.

FIG. 1A, 1B, and 1C separately show a pre-classification type for the morphological identification of the ST segment according to an embodiment of the present invention;
FIG. 2 is a general flow diagram of a method for automatically determining an ST segment according to an embodiment of the present invention;
FIG. 3 is a flow diagram of detecting feature points of the electrocardiogram signal according to an embodiment of the present invention;
FIG. 4 is a flow diagram of comprehensively determining a standard baseline of the electrocardiogram signal according to an embodiment of the present invention;
FIG. 5 is a flow diagram of determining the abnormal changes of the ST segment according to an embodiment of the present invention;
FIG. 6 is a schematic diagram showing the experimental effect of detecting the feature points of the electrocardiogram signal according to an embodiment of the present invention;
FIG. 7 is a schematic diagram showing the experimental effect of removing the average baseline of the electrocardiogram signal according to an embodiment of the present invention;
FIG. 8 is a schematic diagram showing the experimental effect of extracting the ST segment and each baseline segment of the electrocardiogram signal according to an embodiment of the present invention;
FIG. 9 is a flow schematic diagram of an AI-based method for automatically determining the ST segment of the electrocardiogram signal according to an embodiment of the present invention; and
FIG. 10 is a schematic diagram showing the structure of an AI-based device for automatically determining the ST segment of the electrocardiogram signal according to an embodiment of the present invention.

**DETAILED DESCRIPTION OF THE EMBODIMENTS**

The present invention will be further described in conjunction with the drawings and embodiments. It should be noted that as long as there is no conflict, the following embodiments or technical features can be combined to form new embodiments.

Embodiment 1:

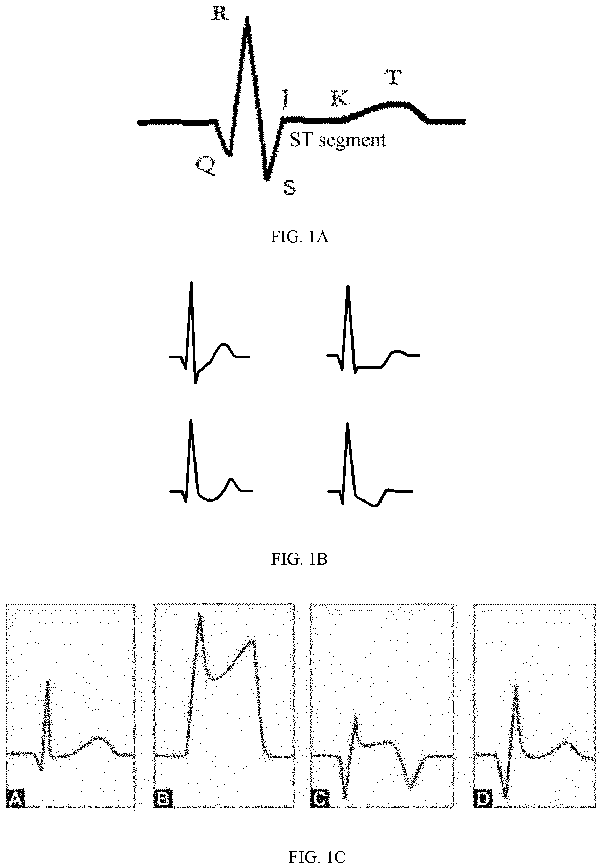

As shown in FIG. 1A, FIG. 1B, and FIG. 1C, in the embodiment of the present invention, the judgment result of the ST segment is divided into three categories, including normal level, elevation, and depression. FIG. 1A shows that the ST segment is normal; FIG. 1B shows that the ST segment is depressed; and FIG. 1C shows that the ST segment is elevated.

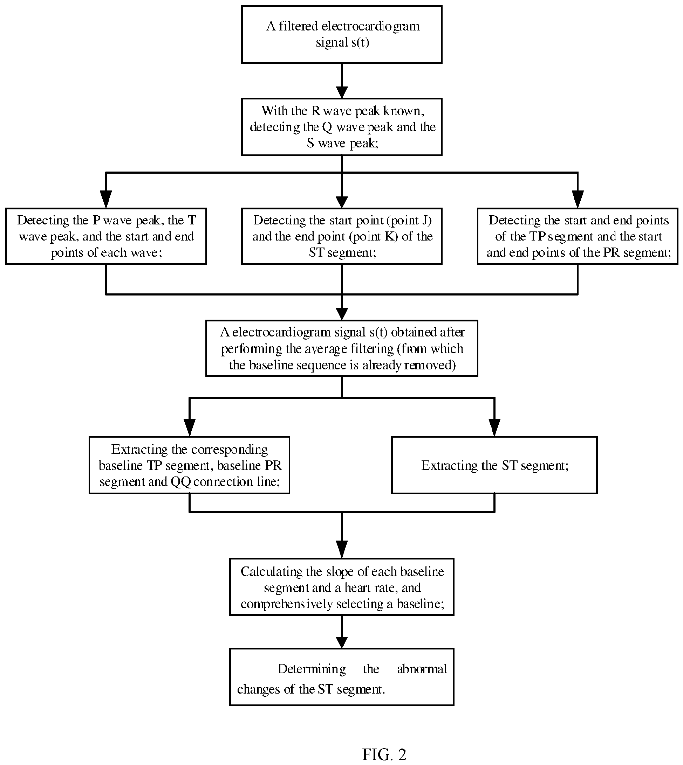

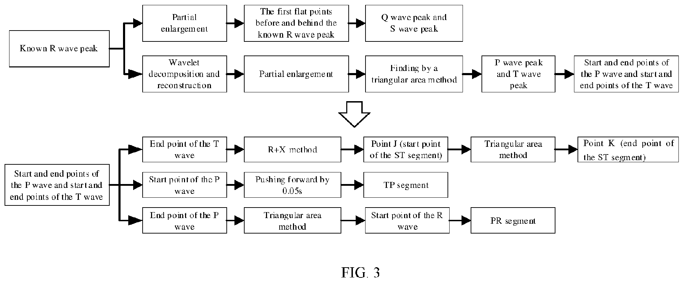

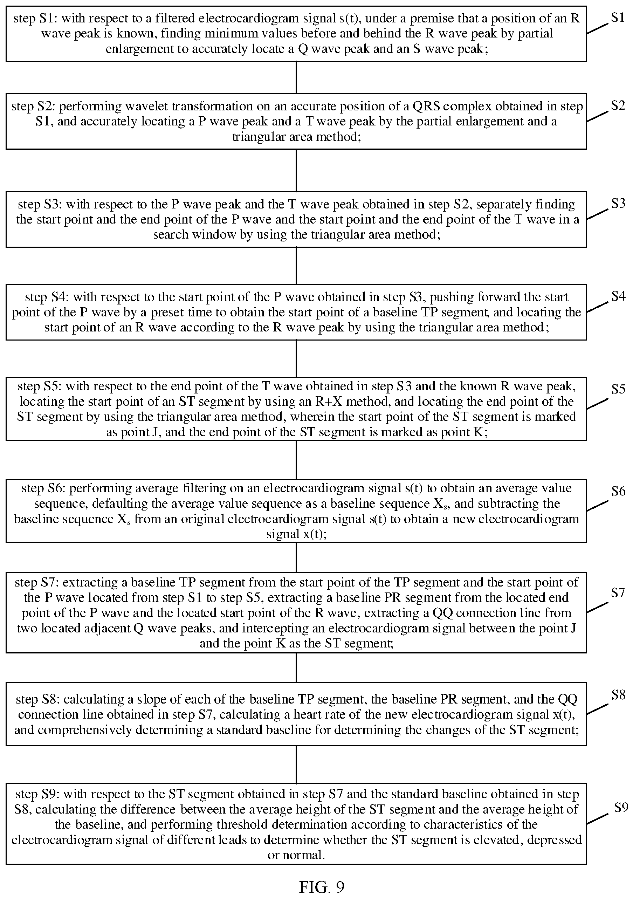

As shown in FIG. 2 and FIG. 9, an embodiment of the present invention provides an AI-based method for automatically determining an ST segment of an electrocardiogram signal, which includes the following steps:
Step S1: with respect to the filtered electrocardiogram signal s(t), under the premise that the position of the R wave peak is known, the minimum values are found before and behind the R wave peak by partial enlargement to accurately locate the Q wave peak and the S wave peak. Specifically, the desensitized electrocardiogram data is taken as the electrocardiogram signal s(t) to be processed, and the electrocardiogram signal is filtered to contain the mark point K $R_{peak}$ of the R wave peak. Therefore, under this premise, based on the relationship of the feature points of the QRS complex, as shown in FIG. 3, using the R wave peak as the standard point, after the 5-layer decomposition and reconstruction of db4 wavelet, the minimum value points are found within 0.5ms before and behind the $R_{peak}$, respectively, which are the Q wave peak $Q_{peak}$ and the S wave peak $S_{peak}$, respectively.

Step S2: wavelet transformation is performed on the accurate position of the QRS complex obtained in step S1, and the P wave peak and the T wave peak are accurately located by partial enlargement and the triangular area method. Specifically, heart beat segmentation is performed on s(t), and each heart beat x(t) is partially enlarged through wavelet decomposition and reconstruction, and then the P wave peak $P_{peak}$ and the T wave peak $T_{peak}$ are obtained by

using the triangular area method. The heart beat segmentation is implemented as: taking $R_{peak}$ as the standard point, pushing forward 0.3s and pushing back 0.5s to intercept the electrocardiogram signal as a single heart beat. Then, wavelet decomposition and reconstruction is performed on the heart beat signal to find the maximum values in the xx window, which are $P_{peak}$ and $T_{peak}$, respectively.

In an embodiment of the present invention, the triangular area method may be specifically implemented as follows: the point 80ms before the A wave peak is taken as the point B, the point X is the variable vertex on the AB trajectory, when the area $S_{\triangle ABX}$ of the triangle reaches the maximum value, the obtained vertex $X'$ correspond to the start point of the A wave peak. Similarly, when the point 80ms after the A wave peak is taken as the point B, the obtained vertex $X'$ is the end point of the A wave peak. This method is well-known to those skilled in the art, and will not be repeated here.

Step S3: with respect to the P wave peak and the T wave peak obtained in step S2, the start point and the end point of the P wave and the start point and the end point of the T wave are separately found in a search window by using the triangular area method. Specifically, the start point $P_{on}$ of the P wave, the start point $T_{on}$ of the T wave, the end point $P_{end}$ of the P wave, and the end point $T_{end}$ of the T wave are found by the triangular area method.

Step S4: with respect to the start point of the P wave obtained in step S3, the start point of the P wave is pushed forward by a preset time to obtain the start point of the baseline TP segment, and the start point of the R wave is located according to the R wave peak by using the triangular area method. Specifically, the start point of the P wave is pushed forward by 0.05s to obtain the start point $TP_{on}$ of the TP segment, and the start point $R_{on}$ of the R wave is located according to $R_{peak}$ by using the triangular area method.

Step S5: with respect to the end point of the T wave obtained in step S3 and the known R wave peak, the start point of the ST segment is located by using the R+X method and is marked as the point J; then the end point of the ST segment is located by using the triangular area method and is marked as the point K. Specifically, with respect to the end point of the T wave obtained in step S3 and the known R wave peak, the start point (the point J) of the ST segment is located by using the R+X method, and then the end point (the point K) of the ST segment is located by using the triangular area method.

The R+X method may be implemented as follows:

According to different heart rates, the position separated behind $R_{peak}$ by n times RT interval is taken as the point J, that is, the calculation formula for the position STi of the point J is:

$$ST_i = R_i + x = \begin{cases} R_i + 0.4RT_i & HR < 100bpm \\ R_i + 0.45RT_i & 100bpm \leq HR < 110bpm \\ R_i + 0.5RT_i & 110bpm \leq HR < 120bpm \\ R_i + 0.55RT_i & HR \geq 120bpm \end{cases}$$

wherein, $R_i$ is the position of the R wave peak; $x$ is a variable related to the heart rate HR; $RT_i$ is the RT interval.

Step S6: average filtering is performed on the electrocardiogram signal s(t) to obtain an average value sequence, and the average value sequence is defaulted as the baseline sequence $X_s$; the baseline sequence $X_s$ is subtracted from the original electrocardiogram signal s(t) to obtain the new electrocardiogram signal x(t).

Step S7: the baseline TP segment is extracted from the start point of the TP segment and the start point of the P wave located in step S1 to step S5; the baseline PR segment is extracted from the located end point of the P wave and the located start point of the R wave; the QQ connection line is extracted from the two located adjacent Q wave peaks; the electrocardiogram signal between the point J and the point K is intercepted as the ST segment. Specifically, with respect to the $TP_{on}$ and $P_{on}$ located in step S1 to step S5, the electrocardiogram signal between $TP_{on}$ and $P_{on}$ is intercepted as the baseline TP segment; with respect to the located $P_{end}$ and $R_{on}$, the electrocardiogram signal between $P_{end}$ and $R_{on}$ is intercepted as the baseline PR segment; a line connecting the located $Q_{peak}$ and the adjacent $Q_{peak}$ is extracted as the QQ connection line; then, with respect to the point J and the point K, the electrocardiogram signal between the point J and the point K is intercepted as the ST segment.

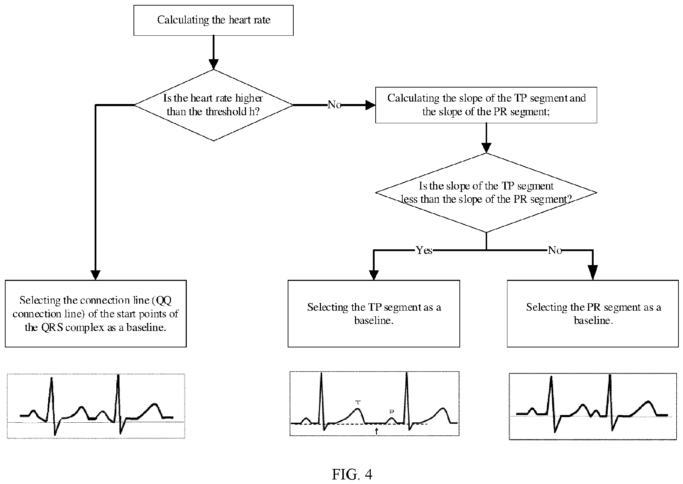

Step S8: as shown in FIG. 4, the slope of each of the baseline TP segment, the baseline PR segment and the QQ connection line obtained in step S7 is calculated, and the heart rate of the new electrocardiogram signal x(t) is calculated; and a standard baseline for determining the change of the ST segment is comprehensively determined. Specifically, with respect to each of the baseline TP segment, the baseline PR segment and the QQ connection line obtained in step S7, the slope $K_{TP}$ and the slope $K_{PR}$ are calculated, the heart rate of the new electrocardiogram signal x(t) is calculated, and a standard baseline for determining the change of the ST segment is comprehensively determined.

The calculation method of the heart rate may be implemented as follows:

$R_{peak}$ is known, the RR interval value RR(i) is first classified and calculated, and then the heart rate HR is obtained, as follows:

$$\begin{cases} RR(i) = R_{\text{peak}}(i+1) - R_{\text{peak}}(i) & R_{\text{peak}}(i+1) > R_{\text{peak}}(i) & i < K \\ RR(i) = 0.8 \times fs & R_{\text{peak}}(i+1) < R_{\text{peak}}(i) & i < K \\ RR(i) = RR(i-1) & i = K \\ HR = 60 \times fs / RR(i) & K \neq 0 \end{cases}$$

wherein, $i$ is the $i^{th}$ heart beat, $K$ is the length of $R_{loc}$, $R_{loc}=R_{peak}$, and $fs$ is the sampling frequency of the electrocardiogram signal.

The method for comprehensively determining the standard baseline is implemented as follows:
according to the calculated heart rate of the electrocardiogram signal x(t), when the heart rate is greater than the threshold h (h=120), the heart rate is considered to be excessively fast, in this case, the QQ connection line is selected as the standard baseline. When the heart rate is not greater than the threshold h (h=120), the slope $K_{TP}$ is compared with the slope $K_{PR}$, wherein when $K_{TP}$ is less than $K_{PR}$, the TP segment is selected as the standard baseline, otherwise, the PR segment is selected as the standard baseline.

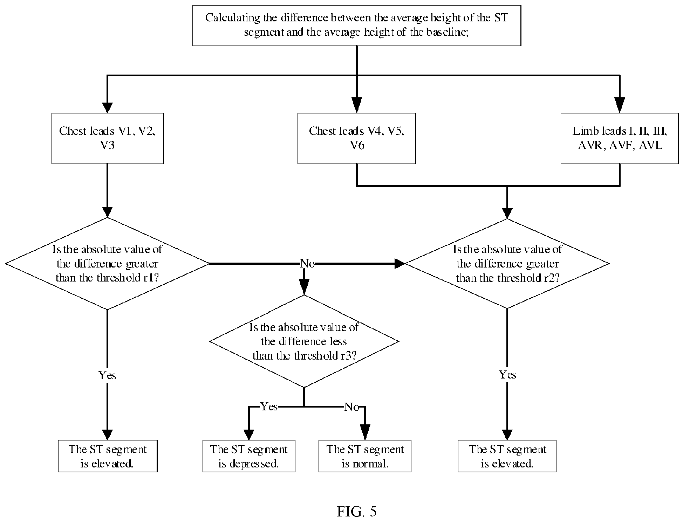

Step S9: as shown in FIG. 5, with respect to the ST segment obtained in step S7 and the standard baseline obtained in step S8, the difference between the average height of the ST segment and the average height of the baseline is calculated; according to the characteristics of the electrocardiogram signal of different leads, threshold determination is performed to determine whether the ST segment is elevated, depressed or normal. Specifically, with respect to the ST segment obtained in step S7 and the standard baseline obtained in step S8, the difference $\Delta H$ between the average height $H_{ST}$ of the ST segment and the average height $H_{bas}$ of the baseline is calculated, and according to the characteristics of the electrocardiogram signal of different leads, threshold determination is performed to determine whether the ST segment is elevated, depressed or normal. The calculation formula of $\Delta H$ is as follows:

$$\Delta H = |H_{ST} - H_{bas}|$$

According to the calculated difference $\Delta H$, the characteristics of the electrocardiogram signal of different leads are taken into consideration as follows:

with respect to limb leads I, II, III, AVR, AVF, AVL and chest leads V4, V5, V6, when $\Delta H$ is greater than the threshold r2 (r2=0.1), it is determined that the ST segment of the electrocardiogram signal of the lead is elevated; when $\Delta H$ is less than the threshold r3 (r3=0.05), it is determined that the ST segment of the electrocardiogram signal of the lead is depressed; otherwise, it is determined that the ST segment of the electrocardiogram signal of the lead is normal; and
with respect to chest leads V1, V2, V3, when $\Delta H$ is greater than the threshold r1 (r1=0.3), it is determined that the ST segment of the electrocardiogram signal of the lead is elevated; when $\Delta H$ is less than the threshold r3 (r3=0.05), it is determined that the ST segment of the electrocardiogram signal of the lead is depressed; otherwise, it is determined that the ST segment of the electrocardiogram signal of the lead is normal.

In order to reduce the error caused by the baseline drift during the extraction of the baseline of the electrocardiogram signal, according to an embodiment of the present invention, after each feature point is extracted, the moving average filter is implemented by performing average filtering to remove the baseline drift. This process is equivalent to low-pass filtering. It has a better effect than the analog filter. Preferably, in the step S6, the method of subtracting the baseline sequence based on the average filtering adopts a moving average filtering function.

Preferably, the moving average filtering function is expressed as follows:

$$X_s(i) = \frac{1}{2N+1}(s(i+N) + s(i+N-1) + ... + s(i-N));$$

wherein, $X_s(i)$ is the $i^{th}$ sampling obtained after smoothing the original electrocardiogram signal s(t); s(i) is the $i^{th}$ sampling point of the original electrocardiogram signal; N is the number of adjacent sampling points and is a positive integer, and N<0.05×sampling frequency.

The data is smoothed by replacing each sampling point with the average of N adjacent sampling points to obtain the baseline sequence $X_s$. The advantage is that the original electrocardiogram signal s(t) is subtracted by $X_s$ to obtain the new electrocardiogram signal x(t), so as to further extract the ST segment, extract the baseline and determine the abnormal changes.

The embodiment of the present invention does not limit the value of N, and preferably, N=5.

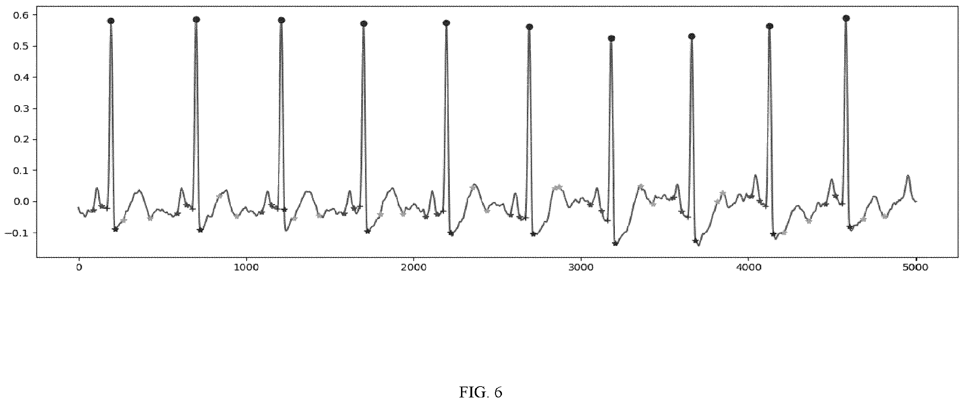

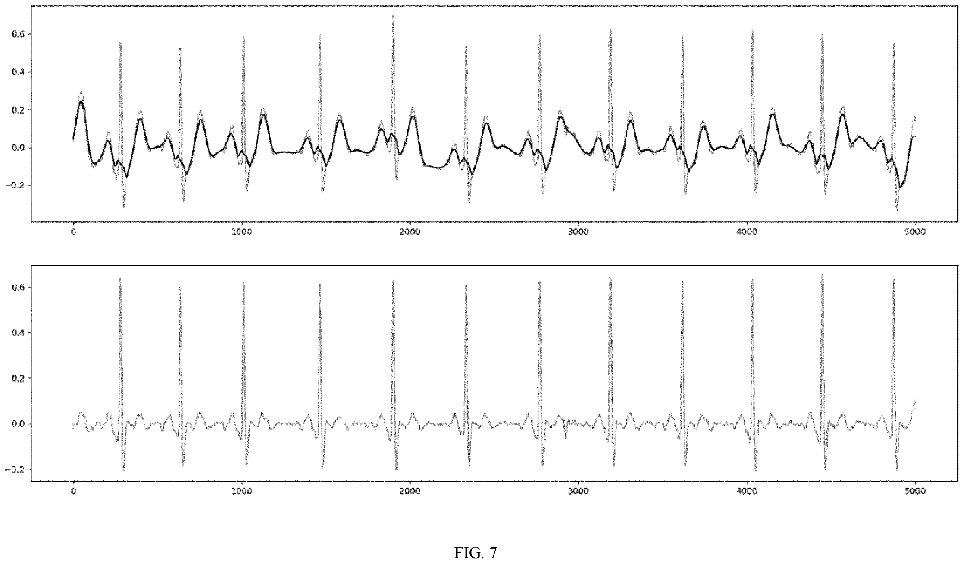

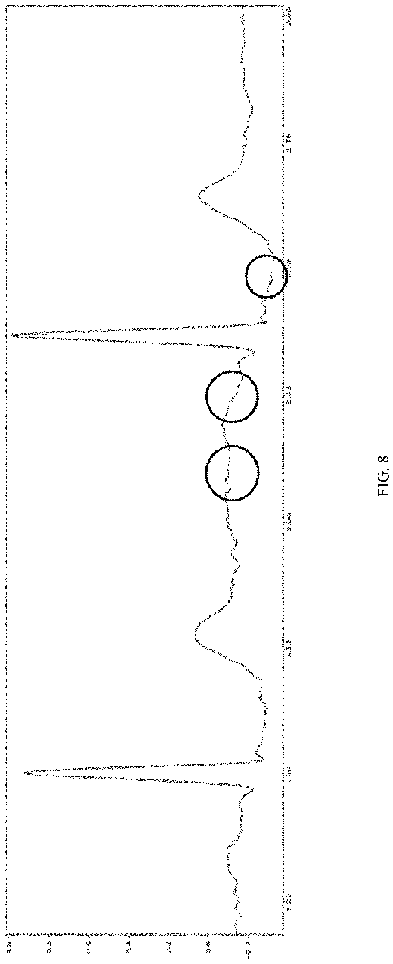

FIG. 6 is a schematic diagram showing the experimental effect of detecting the feature points of the electrocardiogram signal according to an embodiment of the present invention. FIG. 7 is a schematic diagram showing the experimental effect of removing the average baseline of the electrocardiogram signal according to an embodiment of the present invention. FIG. 8 is a schematic diagram showing the experimental effect of extracting the ST segment and each baseline segment of the electrocardiogram signal according to an embodiment of the present invention.

In an embodiment of the present invention, with respect to the filtered human electrocardiogram signal, the key feature points of the electrocardiogram signal are first separately extracted by combining the wavelet filtering and the triangular area method according to the relationship between the various feature points of the electrocardiogram signal. Under the premise that the R wave peak is known, the QRS complex, the P wave, the T wave, the start and end points of each wave, the start and end points of the baseline, and the start and end points of the ST segment are detected, and the start point and the slope characteristic of the ST segment are accurately identified. According to the characteristic of the baseline drift of the electrocardiogram signal, the average filtering is performed on the electrocardiogram signal to remove the baseline sequence to obtain a new electrocardiogram signal. With respect to the new electrocardiogram signal, the ST segment and each baseline segment are extracted from the feature points extracted before the average filtering, and the heart rate and the slope of each baseline segment are calculated, and a standard baseline is determined by considering the heart rate and the slope comprehensively. Finally, the ST segment is compared with the standard baseline according to the amplitude characteristics of the ST segment in different leads, and the abnormal changes of the ST segment are identified based on the standard baseline to obtain the qualitative and quantitative determination result of the ST segment whether it is elevated, depressed, or normal.

According to an embodiment of the present invention, the feature points are first accurately located based on the original signal, and the ST segment and the baseline are extracted after removing the baseline sequence. Moreover, the standard baseline is comprehensively determined, which takes the inaccurate error during the extraction of the baseline caused by the baseline drift as well as other factors such as the heart rate, which influences the baseline selection into consideration. In this way, the present invention solves the problems of the baseline error caused by the baseline drift, the inaccurate selection of the standard baseline, and the large difference in the number of abnormal heart beats, thereby indirectly improving the accuracy of the determination of the abnormal changes of the ST segment. The present invention has a simple calculation, can be easily implemented, and thus provides a new way for rapid and effective identification of the electrocardiogram signals.

The Embodiment 1 mentioned above provides the AI-based method for automatically determining an ST segment of an electrocardiogram signal. Correspondingly, the present invention further provides an AI-based device for automatically determining an ST segment of an electrocardiogram signal. Since the embodiment of the device is generally similar to the embodiment of the method, the description about the embodiment of the device is relatively simple, and the relevant parts can refer to the parts of the description of the embodiment of the method. The following description about the embodiment of the device is merely illustrative.

Embodiment 2:

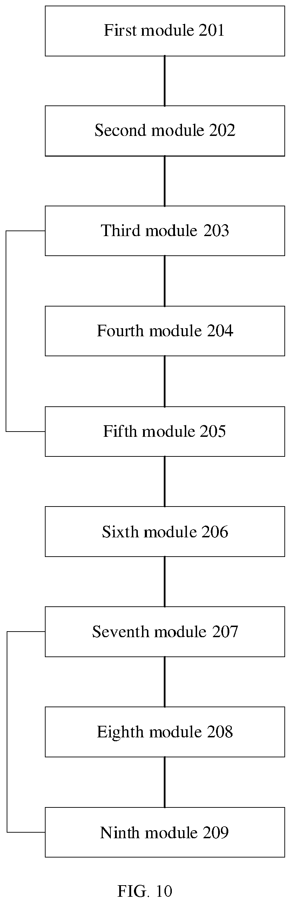

As shown in FIG. 10, an embodiment of the present invention provides an AI-based device for automatically determining an ST segment of an electrocardiogram signal, including:

the first module 201, wherein, with respect to the filtered electrocardiogram signal s(t), under the premise that the position of the R wave peak is known, the first module 201 is configured to find the minimum values before and behind the R wave peak by partial enlargement to accurately locate the Q wave peak and the S wave peak;

the second module 202, wherein, the second module 202 is configured to perform wavelet transformation on the accurate position of the QRS complex obtained in the first module 201, and accurately locate the P wave peak and the T wave peak by partial enlargement and the triangular area method;

the third module 203, wherein, with respect to the P wave peak and the T wave peak obtained in the second module 202, the third module 203 is configured to separately find the start point and the end point of the P wave and the start point and the end point of the T wave in a search window by using the triangular area method;

the fourth module 204, wherein, with respect to the start point of the P wave obtained in the third module 203, the fourth module 204 is configured to push forward the start point of the P wave by a preset time to obtain the start point of the baseline TP segment, and locate the start point of the R wave according to the R wave peak by using the triangular area method;

the fifth module 205, wherein, with respect to the end point of the T wave obtained in the third module 203 and the known R wave peak, the fifth module 205 is configured to locate the start point of the ST segment by using the R+X method, and locate the end point of the ST segment by using the triangular area method, wherein the start point of the ST segment is marked as the point J, and the end point of the ST segment is marked as the point K;

the sixth module 206, wherein the sixth module 206 is configured to perform average filtering on the electrocardiogram signal s(t) to obtain an average value sequence, default the average value sequence as the baseline sequence $X_s$, and subtract the baseline sequence $X_s$ from the original electrocardiogram signal s(t) to obtain the new electrocardiogram signal x(t);

the seventh module 207, wherein the seventh module 207 is configured to extract the baseline TP segment from the start point of the TP segment and the start point of the P wave located from the first module 201 to the fifth module 205, extract the baseline PR segment from the located end point of the P wave and the located start point of the R wave, extract the QQ connection line from the two located adjacent Q wave peaks, and intercept the electrocardiogram signal between the point J and the point K as the ST segment;

the eighth module 208, wherein the eighth module 208 is configured to calculate the slope of each of the baseline TP segment, the baseline PR segment, and the QQ connection line obtained in the seventh module 207, calculate the heart rate of the new electrocardiogram signal x(t), and comprehensively determine a standard baseline for determining the changes of the ST segment, wherein in the eighth module 208, a method for comprehensively determining the standard baseline includes:

calculating the heart rate of the electrocardiogram signal x(t);
when the heart rate is greater than 120, considering the heart rate to be excessively fast, and selecting the QQ connection line as the standard baseline; and
when the heart rate is not greater than 120, calculating the slope $K_{TP}$ of the TP segment and the slope $K_{PR}$ of the PR segment; and when $K_{TP}<K_{PR}$, selecting the TP segment as the standard baseline, otherwise, selecting the PR segment as the standard baseline; and
the ninth module 209, wherein, with respect to the ST segment obtained in the seventh module 207 and the standard baseline obtained in the eighth module 208, the ninth module 209 is configured calculate the difference between the average height of the ST segment and the average height of the baseline, and perform threshold determination according to the characteristics of the electrocardiogram signal of different leads to determine whether the ST segment is elevated, depressed or normal.

Preferably, in the sixth module 206, the method of subtracting the baseline sequence based on the average filtering adopts a moving average filtering function.

Preferably, the moving average filtering function is expressed as follows:

$$X_s(i) = \frac{1}{2N+1}(s(i+N)+s(i+N-1)+...+s(i-N))$$

wherein, $X_s(i)$ is the $i^{th}$ sampling obtained after smoothing the original electrocardiogram signal s(t); s(i) is the $i^{th}$ sampling point of the original electrocardiogram signal; N is the number of adjacent sampling points, and is a positive integer, and N<0.05×sampling frequency.

The data is smoothed by replacing each sampling point with the average of N adjacent sampling points to obtain the baseline sequence $X_s$.

Preferably, N=5.

In an embodiment of the present invention, with respect to the filtered human electrocardiogram signal, the key feature points of the electrocardiogram signal are first separately extracted by combining the wavelet filtering and the triangular area method according to the relationship between the various feature points of the electrocardiogram signal. Under the premise that the R wave peak is known, the QRS complex, the P wave, the T wave, the start and end points of each wave, the start and end points of the baseline, and the start and end points of the ST segment are detected, and the start point and the slope characteristic of the ST segment are accurately identified. According to the characteristic of the baseline drift of the electrocardiogram signal, the average filtering is performed on the electrocardiogram signal to remove the baseline sequence to obtain a new electrocardiogram signal. With respect to the new electrocardiogram signal, the ST segment and each baseline segment are extracted from the feature points extracted before the average filtering, and the heart rate and the slope of each baseline segment are calculated, and a standard baseline is determined by considering the heart rate and the slope comprehensively. Finally, the ST segment is compared with the standard baseline according to the amplitude characteristics of the ST segment in different leads, and the abnormal changes of the ST segment are identified based on the standard baseline to obtain the qualitative and quantitative determination result of the ST segment whether it is elevated, depressed, or normal.

According to an embodiment of the present invention, the feature points are first accurately located based on the original signal, and the ST segment and the baseline are extracted after removing the baseline sequence. Moreover, the standard baseline is comprehensively determined, which takes the inaccurate error during the extraction of the baseline caused by the baseline drift as well as other factors such as the heart rate which influences the baseline selection into consideration. In this way, the present invention solves the problems of the baseline error caused by the baseline drift, the inaccurate selection of the standard baseline, and the large difference in the number of abnormal heart beats, thereby indirectly improving the accuracy of the determination of the abnormal changes of the ST segment. The present invention has a simple calculation, can be easily implemented, and thus provides a new way for rapid and effective identification of the electrocardiogram signals.

On the basis of the prior art, the present invention performs data processing on the existing electrocardiogram signal to determine whether the ST segment of the electrocardiogram signal is elevated, depressed or normal. Obviously, the present invention only relates to direct data processing of the electrocardiogram signal, and does not include the diagnosis and treatment of diseases.

The above description and drawings of the present invention are only the preferred embodiments of the present invention, and do not limit the present invention, which is defined solely by the appended claims.

It should be noted that the embodiments in the present invention as well as the features in the embodiments can be combined with each other as long as there is no conflict.

## Claims

1. An artificial intelligence (AI)-based method for automatically determining an ST segment of an electrocardiogram signal, comprising:

   step S1: with respect to a filtered electrocardiogram signal s(t), under a premise that a position of an R wave peak is known, finding minimum values before and behind the R wave peak by partial enlargement to accurately locate a Q wave peak and an S wave peak;

   step S2: performing wavelet transformation on an accurate position of a QRS complex obtained in step S1, and accurately locating a P wave peak and a T wave peak by the partial enlargement and a triangular area method;

   step S3: with respect to the P wave peak and the T wave peak obtained in step S2, separately finding a start point and an end point of the P wave and a start point and an end point of the T wave in a search window by using the triangular area method;

   step S4: with respect to the start point of the P wave obtained in step S3, pushing forward the start point of the P wave by a preset time to obtain a start point of a baseline TP segment, and locating a start point of an R wave according to the R wave peak by using the triangular area method;

   step S5: with respect to the end point of the T wave obtained in step S3 and the known R wave peak, locating a start point of an ST segment by using an R+X method, and locating an end point of the ST segment by using the triangular area method, wherein the start point of the ST segment is marked as a point J, and the end point of the ST segment is marked as a point K;

   step S6: performing average filtering on an electrocardiogram signal s(t) to obtain an average value sequence, defaulting the average value sequence as a baseline sequence $X_s$, and subtracting the baseline sequence $X_s$ from an original electrocardiogram signal s(t) to obtain a new electrocardiogram signal x(t);

   step S7: extracting a baseline TP segment from the start point of the TP segment and the start point of the P wave located from step S1 to step S5, extracting a baseline PR segment from the located end point of the P wave and the located start point of the R wave, extracting a QQ connection line from two located adjacent Q wave peaks, and intercepting an electrocardiogram signal between the point J and the point K as the ST segment;

   step S8: calculating a slope of each of the baseline TP segment, the baseline PR segment, and the QQ connection line obtained in step S7, calculating a heart rate of the new electrocardiogram signal x(t), and comprehensively determining a standard baseline for determining changes of the ST segment wherein a method for comprehensively determining the standard baseline comprises:

      calculating the heart rate of the electrocardiogram signal x(t);
      when the heart rate is greater than 120, considering the heart rate to be excessively fast, and selecting the QQ connection line as the standard baseline; and
      when the heart rate is not greater than 120, calculating the slope $K_{TP}$ of the TP segment and the slope $K_{PR}$ of the PR segment; when $K_{TP} < K_{PR}$, selecting the TP segment as the standard baseline, otherwise, selecting the PR segment as the standard baseline; and

   step S9: with respect to the ST segment obtained in step S7 and the standard baseline obtained in step S8, calculating a difference between an average height of the ST segment and an average height of the baseline, and performing threshold determination according to characteristics of the electrocardiogram signal of different leads to determine whether the ST segment is elevated, depressed or normal.

2. The AI-based method for automatically determining the ST segment of the electrocardiogram signal according to claim 1, wherein, in step S6, a method of subtracting the baseline sequence based on the average filtering adopts a moving average filtering function.

3. The AI-based method for automatically determining the ST segment of the electrocardiogram signal according to claim 2, wherein the moving average filtering function is expressed as follows:

$$\mathrm{X}_s(i) = \frac{1}{2N+1}(s(i+N)+s(i+N-1)+...+s(i-N))$$

wherein, $\mathrm{X}_s(i)$ is an $i^{th}$ sampling obtained after smoothing an original electrocardiogram signal s(t); s(i) is an $i^{th}$ sampling point of the original electrocardiogram signal; N is a number of adjacent sampling points and is a positive integer, and N<0.05×sampling frequency;
wherein, data is smoothed by replacing each sampling point with an average of N adjacent sampling points to obtain the baseline sequence $\mathrm{X}_s$.

4. The AI-based method for automatically determining the ST segment of the electrocardiogram signal according to claim 3, wherein N=5.

5. An AI-based device for automatically determining an ST segment of an electrocardiogram signal, comprising:

a first module (201), wherein, with respect to a filtered electrocardiogram signal s(t), under a premise that a position of an R wave peak is known, the first module (201) is configured to find minimum values before and behind the R wave peak by partial enlargement to accurately locate a Q wave peak and an S wave peak;
a second module (202), wherein the second module (202) is configured to perform wavelet transformation on an accurate position of a QRS complex obtained in the first module (201,) and accurately locate a P wave peak and a T wave peak by the partial enlargement and a triangular area method;
a third module (203,) wherein, with respect to the P wave peak and the T wave peak obtained in the second module (202), the third module (203) is configured to separately find a start point and an end point of the P wave and a start point and an end point of the T wave in a search window by using the triangular area method;
a fourth module (204,) wherein, with respect to the start point of the P wave obtained in the third module (203), the fourth module (204) is configured to push forward the start point of the P wave by a preset time to obtain a start point of a baseline TP segment, and locate a start point of an R wave according to the R wave peak by using the triangular area method;
a fifth module (205), wherein, with respect to the end point of the T wave obtained in the third module (203) and the known R wave peak, the fifth module (205) is configured to locate a start point of an ST segment by using an R+X method, and locate an end point of the ST segment by using the triangular area method, wherein the start point of the ST segment is marked as a point J, and the end point of the ST segment is marked as a point K;
a sixth module (206), wherein the sixth module (206) is configured to perform average filtering on an electrocardiogram signal s(t) to obtain an average value sequence, default the average value sequence as a baseline sequence $\mathrm{X}_s$, and subtract the baseline sequence $\mathrm{X}_s$ from an original electrocardiogram signal s(t) to obtain a new electrocardiogram signal x(t);
a seventh module (207), wherein the seventh module (207) is configured to extract a baseline TP segment from the start point of the TP segment and the start point of the P wave located from the first module (201) to the fifth module (205), extract a baseline PR segment from the located end point of the P wave and the located start point of the R wave, extract a QQ connection line from two located adjacent Q wave peaks, and intercept an electrocardiogram signal between the point J and the point K as the ST segment;
an eighth module (208), wherein the eighth module (208) is configured to calculate a slope of each of the baseline TP segment, the baseline PR segment, and the QQ connection line obtained in the seventh module (207), calculate a heart rate of the new electrocardiogram signal x(t), and comprehensively determine a standard baseline for determining changes of the ST segment wherein a method for comprehensively determining the standard baseline comprises:
calculating the heart rate of the electrocardiogram signal x(t);
when the heart rate is greater than 120, considering the heart rate to be excessively fast, and selecting the QQ connection line as the standard baseline; and

when the heart rate is not greater than 120, calculating the slope $K_{TP}$ of the TP segment and the slope $K_{PR}$ of the PR segment; when $K_{TP}<K_{PR}$, selecting the TP segment as the standard baseline, otherwise, selecting the PR segment as the standard baseline; and

a ninth module (209), wherein, with respect to the ST segment obtained in the seventh module (207) and the standard baseline obtained in the eighth module (208), the ninth module (209) is configured calculate a difference between an average height of the ST segment and an average height of the baseline, and perform threshold determination according to characteristics of the electrocardiogram signal of different leads to determine whether the ST segment is elevated, depressed or normal.

6. The AI-based device for automatically determining the ST segment of the electrocardiogram signal according to claim 5, wherein, in the sixth module, a method of subtracting the baseline sequence based on the average filtering adopts a moving average filtering function.

7. The AI-based device for automatically determining the ST segment of the electrocardiogram signal according to claim 6, wherein the moving average filtering function is expressed as follows:

$$\mathbf{X}_s(i) = \frac{1}{2N+1}(s(i+N) + s(i+N-1) + ... + s(i-N))$$

wherein, $X_s(i)$ is an $i^{th}$ sampling obtained after smoothing an original electrocardiogram signal s(t); s(i) is an $i^{th}$ sampling point of the original electrocardiogram signal; N is a number of adjacent sampling points and is a positive integer, and $N<0.05\times$sampling frequency;

wherein, data is smoothed by replacing each sampling point with an average of N adjacent sampling points to obtain the baseline sequence $X_s$.

8. The AI-based device for automatically determining the ST segment of the electrocardiogram signal according to claim 7, wherein N=5.

**Patentansprüche**

1. Auf künstlicher Intelligenz (KI)-basiertes Verfahren zum automatischen Bestimmen eines ST-Segments eines Elektrokardiogrammsignals, umfassend:

Schritt S1: in Bezug auf ein gefiltertes Elektrokardiogrammsignal s(t) unter der Annahme, dass eine Position einer R-Wellenspitze bekannt ist, Ermitteln von Minimumwerten vor und nach der R-Wellenspitze durch Teilvergrößerung, um eine Q-Wellenspitze und eine S-Wellenspitze präzise zu lokalisieren;

Schritt S2: Durchführen einer Wavelet-Transformation an einer präzisen Position eines QRS-Komplexes, der in Schritt S1 erlangt wurde, und durch Teilvergrößerung und ein Dreiecksbereichverfahren präzises Lokalisieren einer P-Wellenspitze und einer T-Wellenspitze;

Schritt S3: in Bezug auf die P-Wellenspitze und die T-Wellenspitze, die in Schritt S2 erlangt wurden, mittels des Dreiecksbereichverfahrens separates Ermitteln eines Anfangspunkts und eines Endpunkts der P-Welle und eines Anfangspunkts und eines Endpunkts der T-Welle in einem Suchfenster;

Schritt S4: in Bezug auf den Anfangspunkt der P-Welle, der in Schritt S3 erlangt wurde, Verschieben des Anfangspunkts der P-Welle um eine im Voraus festgelegte Zeit nach vorne, um einen Anfangspunkt eines Grundlinien-TP-Segments zu erlangen, und mittels des Dreiecksbereichverfahrens Lokalisieren eines Anfangspunkts einer R-Welle gemäß der R-Wellenspitze;

Schritt S5: in Bezug auf den Endpunkt der T-Welle, der in Schritt S3 erlangt wurde, und der bekannten R-Wellenspitze Lokalisieren eines Anfangspunkts eines ST-Segments unter Verwendung eines R+X-Verfahrens und Lokalisieren eines Endpunkts des ST-Segments unter Verwendung des Dreiecksbereichverfahrens, wobei der Anfangspunkt des ST-Segments als ein Punkt J markiert wird und der Endpunkt des ST-Segments als ein Punkt K markiert wird;

Schritt S6: Durchführen einer Mittelwertfilterung an einem Elektrokardiogrammsignal s(t), um eine Mittelwertsequenz zu erlangen, standardmäßiges Anwenden der Mittelwertsequenz als Grundliniensequenz $X_s$ und Subtrahieren der Grundliniensequenz $X_s$ von einem ursprünglichen Elektrokardiogrammsignal s(t), um ein neues Elektrokardiogrammsignal x(t) zu erlangen;

Schritt S7: Extrahieren eines Grundlinien-TP-Segments aus dem Anfangspunkt des TP-Segments und dem Anfangspunkt der P-Welle, die in Schritt S1 bis S5 lokalisiert wurden, Extrahieren eines Grundlinie-PR-Segments aus dem lokalisierten Endpunkt der P-Welle und dem lokalisierten Anfangspunkt der R-Welle, Extrahieren einer QQ-Verbindungslinie aus zwei lokalisierten benachbarten Q-Wellenspitzen und Abgreifen eines Elektrokardiogrammsignals zwischen dem Punkt J und dem Punkt K als das ST-Segment;

Schritt S8: Berechnen einer Flanke von jedem des Grundlinien-TP-Segments, des Grundlinien-PR-Segments und der QQ-Verbindungslinie, die in Schritt S7 erlangt wurde, Berechnen einer Herzfrequenz des neuen Elektrokardiogrammsignals x(t) und umfassendes Bestimmen einer Referenzgrundlinie zum Bestimmen von Änderungen des ST-Segments, wobei ein Verfahren zum umfassenden Bestimmen der Referenzgrundlinie umfasst:

Berechnen der Herzfrequenz des Elektrokardiogrammsignals x(t);
wenn die Herzfrequenz größer als 120 ist, Betrachten der Herzfrequenz als übermäßig schnell und Auswählen der QQ-Verbindungslinie als Referenzgrundlinie; und
wenn die Herzfrequenz nicht größer als 120 ist, Berechnen der Flanke $K_{TP}$ des TP-Segments und der Flanke $K_{PR}$ des PR-Segments; wenn $K_{TP} < K_{PR}$, Auswählen des TP-Segments als Referenzgrundlinie, anderenfalls Auswählen des PR-Segments als Referenzgrundlinie; und

Schritt S9: in Bezug auf das ST-Segment, das in Schritt S7 erlangt wurde, und die Referenzgrundlinie, die in Schritt S8 erlangt wurde, Berechnen einer Differenz zwischen einer durchschnittlichen Höhe des ST-Segments und einer durchschnittlichen Höhe der Grundlinie und Durchführen einer Schwellenwertbestimmung gemäß der Kennlinien des Elektrokardiogrammsignals unterschiedlicher Leitungen, um zu bestimmen, ob das ST-Segment erhöht, vertieft oder normal ist.

2. KI-basiertes Verfahren zum automatischen Bestimmen des ST-Segments eines Elektrokardiogrammsignals nach Anspruch 1, wobei in Schritt S6 ein Verfahren des Subtrahierens der Grundliniensequenz auf Grundlage der Mittelwertfilterung eine Filterungsfunktion mit gleitendem Mittelwert anwendet.

3. KI-basiertes Verfahren zum automatischen Bestimmen des ST-Segments eines Elektrokardiogrammsignals nach Anspruch 2, wobei die Filterungsfunktion mit gleitendem Mittelwert wie folgt ausgedrückt wird:

$$X_s(i) = \frac{1}{2N+1}(s(i+N) + s(i+N-1) + \ldots + s(i-N))$$

wobei $X_s(i)$ eine $i$-te Abtastung ist, die nach dem Glätten eines ursprünglichen Elektrokardiogrammsignals s(t) erlangt wurde; s(i) ein $i$-ter Abtastungspunkt des ursprünglichen Elektrokardiogrammsignals ist; N eine Anzahl benachbarter Abtastungspunkte und eine positive Ganzzahl ist und N<0,05 × Abtastfrequenz;
wobei Daten durch Ersetzen jedes Abtastungspunkts mit einem Mittelwert von N benachbarten Abtastungspunkten geglättet werden, um die Grundliniensequenz $X_s$ zu erlangen.

4. KI-basiertes Verfahren zum automatischen Bestimmen des ST-Segments eines Elektrokardiogrammsignals nach Anspruch 3, wobei N=5.

5. KI-basierte Vorrichtung zum automatischen Bestimmen des ST-Segments eines Elektrokardiogrammsignals, umfassend:

ein erste Modul (201), wobei in Bezug auf ein gefiltertes Elektrokardiogrammsignal s(t) unter der Annahme, dass eine Position einer R-Wellenspitze bekannt ist, das erste Modul (201) dazu konfiguriert ist, durch Teilvergrößerung Minimumwerte vor und nach der R-Wellenspitze zu ermitteln, um eine Q-Wellenspitze und eine S-Wellenspitze präzise zu lokalisieren;
ein zweites Modul (202), wobei das zweite Modul (202) dazu konfiguriert ist, eine Wavelet-Transformation an einer präzisen Position eines QRS-Komplexes durchzuführen, der im ersten Modul (201) erlangt wurde, und durch Teilvergrößerung und ein Dreiecksbereichverfahren eine P-Wellenspitze und eine T-Wellenspitze präzise zu lokalisieren;
ein drittes Modul (203), wobei in Bezug auf die P-Wellenspitze und die T-Wellenspitze, die im zweiten Modul (202) erlangt wurden, das dritte Modul (203) dazu konfiguriert ist, mittels des Dreiecksbereichverfahrens separat einen Anfangspunkt und einen Endpunkt der P-Welle und einen Anfangspunkt und einen Endpunkt der T-Welle in einem Suchfenster zu ermitteln;
ein viertes Modul (204), wobei in Bezug auf den Anfangspunkt der P-Welle, der im dritten Modul (203) erlangt wurde, das vierte Modul (204) dazu konfiguriert ist, den Anfangspunkt der P-Welle um eine im Voraus festgelegte Zeit nach vorne zu schieben, um einen Anfangspunkt eines Grundlinien-TP-Segments zu erlangen, und mittels des Dreiecksbereichverfahrens einen Anfangspunkt einer R-Welle gemäß der R-Wellenspitze zu lokalisieren; ein fünftes Modul (205), wobei in Bezug auf den Endpunkt der T-Welle, der im dritten Modul (203) erlangt wurde, und der bekannten R-Wellenspitze das fünfte Modul (205) dazu konfiguriert ist, einen Anfangspunkt eines ST-Segments unter Verwendung eines R+X-Verfahrens zu lokalisieren und einen Endpunkt des ST-Segments unter Verwendung des Dreiecksbereichverfahrens zu lokalisieren, wobei der Anfangspunkt des ST-Segments als ein Punkt J markiert ist und der Endpunkt des ST-Segments als ein Punkt K markiert ist; ein sechste Modul (206), wobei das sechste Modul (206) dazu konfiguriert ist, eine Mittelwertfilterung an einem Elektrokardiogrammsignal s(t) durchzuführen, um eine Mittelwertsequenz zu erlangen, die Mittelwertsequenz standardmäßig als Grundliniensequenz $X_s$ anzuwenden und die Grundliniensequenz $X_s$ von einem ursprünglichen Elektrokardiogrammsignal s(t) zu subtrahieren, um ein neues Elektrokardiogrammsignal x(t) zu erlangen; ein siebtes Modul (207), wobei das siebte Modul (207) dazu konfiguriert ist, ein Grundlinien-TP-Segment aus dem Anfangspunkt des TP-Segments und dem Anfangspunkt der P-Welle, die im ersten Modul (201) bis fünften Modul (205) lokalisiert wurden, zu extrahieren, ein Grundlinie-PR-Segment aus dem lokalisierten Endpunkt der P-Welle und dem lokalisierten Anfangspunkt der R-Welle zu extrahieren, eine QQ-Verbindungslinie aus zwei lokalisierten benachbarten Q-Wellenspitzen zu extrahieren und ein Elektrokardiogrammsignal zwischen dem Punkt J und dem Punkt K als das ST-Segment abzugreifen; ein achtes Modul (208), wobei das achte Modul (208) dazu konfiguriert ist, eine Flanke von jedem des Grundlinien-TP-Segments, des Grundlinien-PR-Segments und der QQ-Verbindungslinie zu berechnen, die im siebten Modul (207) erlangt wurde, eine Herzfrequenz des neuen Elektrokardiogrammsignals x(t) zu berechnen und umfassend eine Referenzgrundlinie zum Bestimmen von Änderungen des ST-Segments zu bestimmen, wobei ein Verfahren zum umfassenden Bestimmen der Referenzgrundlinie umfasst:

Berechnen der Herzfrequenz des Elektrokardiogrammsignals x(t);
wenn die Herzfrequenz größer als 120 ist, Betrachten der Herzfrequenz als übermäßig schnell und Auswählen der QQ-Verbindungslinie als Referenzgrundlinie; und
wenn die Herzfrequenz nicht größer als 120 ist, Berechnen der Flanke $K_{TP}$ des TP-Segments und der Flanke $K_{PR}$ des PR-Segments; wenn $K_{TP}<K_{PR}$, Auswählen des TP-Segments als Referenzgrundlinie, anderenfalls Auswählen des PR-Segments als Referenzgrundlinie; und
ein neuntes Modul (209), wobei in Bezug auf das ST-Segment, das im siebten Modul (207) erlangt wurde, und die Referenzgrundlinie, die im achten Modul (208) erlangt wurde, das neunte Modul (209) dazu konfiguriert ist, eine Differenz zwischen einer durchschnittlichen Höhe des ST-Segments und einer durchschnittlichen Höhe der Grundlinie zu berechnen und eine Schwellenwertbestimmung gemäß der Kennlinien des Elektrokardiogrammsignals unterschiedlicher Leitungen durchzuführen, um zu bestimmen, ob das ST-Segment erhöht, vertieft oder normal ist.

6. KI-basierte Vorrichtung zum automatischen Bestimmen des ST-Segments eines Elektrokardiogrammsignals nach Anspruch 5, wobei im sechsten Modul ein Verfahren des Subtrahierens der Grundliniensequenz auf Grundlage der Mittelwertfilterung eine Filterungsfunktion mit gleitendem Mittelwert anwendet.

7. KI-basierte Vorrichtung zum automatischen Bestimmen des ST-Segments eines Elektrokardiogrammsignals nach Anspruch 6, wobei die Filterungsfunktion mit gleitendem Mittelwert wie folgt ausgedrückt wird:

$$X_s(i) = \frac{1}{2N+1}(s(i+N) + s(i+N-1) + ... + s(i-N))$$

wobei $X_s(i)$ eine i-te Abtastung ist, die nach dem Glätten eines ursprünglichen Elektrokardiogrammsignals s(t) erlangt wurde; s(i) ein i-ter Abtastungspunkt des ursprünglichen Elektrokardiogrammsignals ist; N eine Anzahl benachbarter Abtastungspunkte und eine positive Ganzzahl ist und N<0,05 × Abtastfrequenz;
wobei Daten durch Ersetzen jedes Abtastungspunkts mit einem Mittelwert von N benachbarten Abtastungspunkten geglättet werden, um die Grundliniensequenz $X_s$ zu erlangen.

8. KI-basierte Vorrichtung zum automatischen Bestimmen des ST-Segments eines Elektrokardiogrammsignals nach Anspruch 7, wobei N=5.

**Revendications**

1. Un procédé basé sur une intelligence artificielle (IA) pour déterminer automatiquement un segment ST d'un signal d'électrocardiogramme, comprenant :

   une étape S1 : en rapport avec un signal s(t) d'électrocardiogramme filtré, partant du principe que la position d'un pic d'onde R est connue, trouver les valeurs minimales avant et à la suite du pic d'onde R par agrandissement partiel afin de localiser de façon précise un pic d'onde Q et un pic d'onde S ;
   une étape S2 : réaliser une transformation d'ondelette sur une position précise du complexe QRS obtenue à l'étape S1, et une localisation précise d'un pic d'onde P et d'un pic d'onde T par agrandissement partiel et par procédé de zone triangulaire ;
   une étape S3 : en rapport avec le pic d'onde P et le pic d'onde T obtenus à l'étape S2, trouver séparément un point de départ et un point d'arrivée de l'onde P un point de départ et un point d'arrivée de l'onde T dans une fenêtre de recherche en utilisant le procédé de zone triangulaire ;
   une étape S4 : en rapport avec le point de départ de l'onde P obtenu à l'étape S3, avancer le point de départ de l'onde P à un temps prédéfini afin d'obtenir un point de départ d'un segment TP de ligne de base, et localiser un point de départ d'une onde R en fonction du pic d'onde R en utilisant le procédé de zone triangulaire ;
   une étape S5 : en rapport avec le point d'arrivée de l'onde T obtenu à l'étape S3 et le pic d'onde R connu, localiser le point de départ d'un segment ST en utilisant le procédé R+X, et localiser le point d'arrivée du segment ST en utilisant le procédé de zone triangulaire, dans lequel le point de départ du segment ST est marqué point J, et le point d'arrivée du segment ST est marqué point K;
   une étape S6 : réaliser un filtrage moyen d'un signal s(t) d'électrocardiogramme pour obtenir une séquence de valeur moyenne, définir par défaut la séquence de valeur moyenne comme séquence $X_s$ de ligne de base, et soustraire la séquence $X_s$ de ligne de base au signal s(t) d'électrocardiogramme originel pour obtenir un nouveau signal x(t) d'électrocardiogramme ;
   une étape S7 : extraire le segment TP de ligne de base depuis le point de départ du segment TP et le point de départ de l'onde P localisés de l'étape S1 à l'étape S5, extraire le segment PR de ligne de base depuis le point d'arrivée de l'onde P et le point de départ localisé de l'onde R, extraire une ligne de connexion QQ à partir de deux pics localisés d'onde Q adjacents, et intercepter un signal d'électrocardiogramme entre le point J et le point K comme segment ST ;
   une étape S8 : calculer une pente de chaque segment TP de ligne de base, segment PR de ligne de base, et ligne de connexion QQ obtenus à l'étape S7, calculer un rythme cardiaque du nouveau signal x(t) d'électrocardiogramme, et déterminer entièrement une ligne de base standard pour déterminer les changements du segment ST dans lequel le procédé pour déterminer entièrement la ligne de base standard comprend :

      le calcul du rythme cardiaque du signal x(t) d'électrocardiogramme ;
      lorsque le rythme cardiaque est supérieur à 120, considérer que le rythme cardiaque est excessivement rapide, et sélectionner la ligne de connexion QQ comme ligne de base standard ; et
      lorsque le rythme cardiaque est inférieur à 120, calculer la pente $K_{TP}$ du segment TP et la pente $K_{PR}$ du segment PR ; lorsque $K_{TP}<K_{PR}$, sélectionner le segment TP comme ligne de base standard, à défaut, sélectionner le segment PR comme ligne de base standard ; et

   une étape S9 : en rapport avec le segment ST obtenu à l'étape S7 et la ligne de base standard obtenue à l'étape S8, calculer la différence entre une hauteur moyenne du segment ST et une hauteur moyenne de la ligne de base, et réaliser la détermination du seuil en fonction des caractéristiques du signal d'électrocardiogramme de différentes dérivations afin de déterminer si le segment ST est élevé, enfoncé ou normal.

2. Un procédé basé sur une intelligence artificielle pour déterminer automatiquement un segment ST d'un signal d'électrocardiogramme selon la revendication 1, dans lequel, à l'étape S6, un procédé de soustraction de la séquence de signe de base basé sur le filtrage moyen emploie une fonction de filtrage à moyenne mobile.

3. Un procédé basé sur une intelligence artificielle pour déterminer automatiquement un segment ST d'un signal d'électrocardiogramme selon la revendication 2, dans lequel la fonction de filtrage à moyenne mobile est exprimée comme suit :

$$X_s(i) = \frac{1}{2N+1}(s(i+N) + s(i+N-1) + \ldots + s(i-N))$$

dans lequel, $X_s(i)$ est le i<sup>ème</sup> échantillon obtenu après lissage du signal s(t) d'électrocardiogramme originel ; s(i) est le i<sup>ème</sup> point d'échantillonnage du signal d'électrocardiogramme originel ; N est le nombre de points d'échantillonnage adjacents est un nombre entier positif, et N < 0,05 × fréquence d'échantillonnage ;

dans lequel, les données sont lissées par remplacement de chaque point d'échantillon par une moyenne de N points d'échantillonnage adjacents pour obtenir la séquence $X_s$ de ligne de base.

**4.** Un procédé basé sur une intelligence artificielle pour déterminer automatiquement un segment ST d'un signal d'électrocardiogramme selon la revendication 3, dans lequel N = 5.

**5.** Un dispositif basé sur une intelligence artificielle pour déterminer automatiquement un segment ST d'un signal d'électrocardiogramme, comprenant :

un premier module (201), dans lequel, en rapport avec un signal s(t) d'électrocardiogramme filtré, partant du principe que la position d'un pic d'onde R est connue, le premier module (201) est configuré pour trouver les valeurs minimales avant et à la suite du pic d'onde R par agrandissement partiel afin de localiser de façon précise un pic d'onde Q et un pic d'onde S ;

un deuxième module (202), dans lequel, le deuxième module (202) est configuré pour réaliser une transformation d'ondelette sur une position précise du complexe QRS obtenue dans le premier module (201), et une localisation précise d'un pic d'onde P et d'un pic d'onde T par agrandissement partiel et par procédé de zone triangulaire ;

un troisième module (203), dans lequel, en rapport avec le pic d'onde P et le pic d'onde T obtenus dans le deuxième module (202), le troisième module (203) est configuré pour trouver séparément un point de départ et un point d'arrivée de l'onde P un point de départ et un point d'arrivée de l'onde T dans une fenêtre de recherche en utilisant le procédé de zone triangulaire ;

un quatrième module (204), dans lequel, en rapport avec le point de départ de l'onde P obtenu dans le troisième module (203), le quatrième module (204) est configuré pour avancer le point de départ de l'onde P à un temps prédéfini afin d'obtenir un point de départ d'un segment TP de ligne de base, et localiser un point de départ d'une onde R en fonction du pic d'onde R en utilisant le procédé de zone triangulaire ;

un cinquième module (205), dans lequel, en rapport avec le point d'arrivée de l'onde T obtenu dans le troisième module (203) et le pic d'onde R connu, le cinquième module (205) est configuré pour localiser le point de départ d'un segment ST en utilisant le procédé R+X, et localiser le point d'arrivée du segment ST en utilisant le procédé de zone triangulaire, dans lequel le point de départ du segment ST est marqué point J, et le point d'arrivée du segment ST est marqué point K;

un sixième module (206), dans lequel, dans lequel le sixième module (206) est configuré pour réaliser un filtrage moyen d'un signal s(t) d'électrocardiogramme pour obtenir une séquence de valeur moyenne, définir par défaut la séquence de valeur moyenne comme séquence $X_s$ de ligne de base, et soustraire la séquence $X_s$ de ligne de base au signal s(t) d'électrocardiogramme originel pour obtenir un nouveau signal x(t) d'électrocardiogramme ;

un septième module (207), dans lequel le septième module (207) est configuré pour extraire le segment TP de ligne de base depuis le point de départ du segment TP et le point de départ de l'onde P localisés depuis le premier module (201) jusqu'au cinquième module (205), extraire le segment PR de ligne de base depuis le point d'arrivée de l'onde P et le point de départ localisé de l'onde R, extraire une ligne de connexion QQ à partir de deux pics localisés d'onde Q adjacentes, et intercepter un signal d'électrocardiogramme entre le point J et le point K comme segment ST ;

un huitième module (208) dans lequel le huitième module (208) est configuré pour calculer une pente de chaque segment TP de ligne de base, segment PR de ligne de base, et ligne de connexion QQ obtenus dans le septième module (207), calculer un rythme cardiaque du nouveau signal x(t) d'électrocardiogramme, et déterminer entièrement une ligne de base standard pour déterminer les changements du segment ST dans lequel le procédé pour déterminer entièrement la ligne de base standard comprend :

le calcul du rythme cardiaque du signal x(t) d'électrocardiogramme ;
lorsque le rythme cardiaque est supérieur à 120, considérer que le rythme cardiaque est excessivement rapide, et sélectionner la ligne de connexion QQ comme ligne de base standard ; et
lorsque le rythme cardiaque est inférieur à 120, calculer la pente $K_{TP}$ du segment TP et la pente $K_{PR}$ du segment PR ; lorsque $K_{TP} < K_{PR}$, sélectionner le segment TP comme ligne de base standard, à défaut, sélectionner le segment PR comme ligne de base standard ; et
un neuvième module (209), dans lequel, en rapport avec le segment ST obtenu dans le septième module (207) et la ligne de base standard obtenue dans le huitième module (208), le neuvième module (209) est configuré pour calculer la différence entre une hauteur moyenne du segment ST et une hauteur moyenne de la ligne de base, et réaliser la détermination du seuil en fonction des caractéristiques du signal d'électrocardiogramme de différentes dérivations afin de déterminer si le segment ST est élevé, enfoncé ou normal.

6. Un dispositif basé sur une intelligence artificielle pour déterminer automatiquement un segment ST d'un signal d'électrocardiogramme selon la revendication 5, dans lequel, dans le sixième module, un procédé de soustraction de la séquence de signe de base basé sur le filtrage moyen emploie une fonction de filtrage à moyenne mobile.

7. Un dispositif basé sur une intelligence artificielle pour déterminer automatiquement un segment ST d'un signal d'électrocardiogramme selon la revendication 6, dans lequel la fonction de filtrage à moyenne mobile est exprimée comme suit :

$$X_s(i) = \frac{1}{2N+1}(s(i+N) + s(i+N-1) + \ldots + s(i-N))$$

dans lequel, $X_s(i)$ est le $i^{ème}$ échantillon obtenu après lissage du signal $s(t)$ d'électrocardiogramme originel; $s(i)$ est le $i^{ème}$ point d'échantillonnage du signal d'électrocardiogramme originel ; N est le nombre de points d'échantillonnage adjacents est un nombre entier positif, et N < 0,05 × fréquence d'échantillonnage ;
dans lequel, les données sont lissées par remplacement de chaque point d'échantillon par une moyenne de N points d'échantillonnage adjacents pour obtenir la séquence $X_s$ de ligne de base.

8. Un dispositif basé sur une intelligence artificielle pour déterminer automatiquement un segment ST d'un signal d'électrocardiogramme selon la revendication 7, dans lequel N = 5.

FIG. 1A

FIG. 1B

FIG. 1C

A filtered electrocardiogram signal s(t)

With the R wave peak known, detecting the Q wave peak and the S wave peak;

Detecting the P wave peak, the T wave peak, and the start and end points of each wave;

Detecting the start point (point J) and the end point (point K) of the ST segment;

Detecting the start and end points of the TP segment and the start and end points of the PR segment;

A electrocardiogram signal s(t) obtained after performing the average filtering (from which the baseline sequence is already removed)

Extracting the corresponding baseline TP segment, baseline PR segment and QQ connection line;

Extracting the ST segment;

Calculating the slope of each baseline segment and a heart rate, and comprehensively selecting a baseline;

Determining the abnormal changes of the ST segment.

FIG. 2

| Known R wave peak | → | Partial enlargement | → | The first flat points before and behind the known R wave peak | → | Q wave peak and S wave peak |

| | → | Wavelet decomposition and reconstruction | → | Partial enlargement | → | Finding by a triangular area method | → | P wave peak and T wave peak | → | Start and end points of the P wave and start and end points of the T wave |

| Start and end points of the P wave and start and end points of the T wave | → | End point of the T wave | → | R+X method | → | Point J (start point of the ST segment) | → | Triangular area method | → | Point K (end point of the ST segment) |

| | → | Start point of the P wave | → | Pushing forward by 0.05s | → | TP segment |

| | → | End point of the P wave | → | Triangular area method | → | Start point of the R wave | → | PR segment |

FIG. 3

Calculating the heart rate

Is the heart rate higher than the threshold h?

No

Calculating the slope of the TP segment and the slope of the PR segment;

Is the slope of the TP segment less than the slope of the PR segment?

Yes

No

Selecting the connection line (QQ connection line) of the start points of the QRS complex as a baseline.

Selecting the TP segment as a baseline.

Selecting the PR segment as a baseline.

FIG. 4

Calculating the difference between the average height of the ST
segment and the average height of the baseline;

Chest leads V1, V2,
V3

Chest leads V4, V5,
V6

Limb leads I, II, III,
AVR, AVF, AVL

Is the absolute value of
the difference greater
than the threshold r1?

No

Is the absolute value of
the difference greater
than the threshold r2?

Is the absolute value of
the difference less
than the threshold r3?

Yes

Yes

Yes          No

The ST segment
is elevated.

The ST segment
is depressed.

The ST segment
is normal.

The ST segment
is elevated.

FIG. 5

FIG. 6

FIG. 7

EP 3 795 074 B1

FIG. 8

step S1: with respect to a filtered electrocardiogram signal s(t), under a premise that a position of an R wave peak is known, finding minimum values before and behind the R wave peak by partial enlargement to accurately locate a Q wave peak and an S wave peak; — S1

step S2: performing wavelet transformation on an accurate position of a QRS complex obtained in step S1, and accurately locating a P wave peak and a T wave peak by the partial enlargement and a triangular area method; — S2

step S3: with respect to the P wave peak and the T wave peak obtained in step S2, separately finding the start point and the end point of the P wave and the start point and the end point of the T wave in a search window by using the triangular area method; — S3

step S4: with respect to the start point of the P wave obtained in step S3, pushing forward the start point of the P wave by a preset time to obtain the start point of a baseline TP segment, and locating the start point of an R wave according to the R wave peak by using the triangular area method; — S4

step S5: with respect to the end point of the T wave obtained in step S3 and the known R wave peak, locating the start point of an ST segment by using an R+X method, and locating the end point of the ST segment by using the triangular area method, wherein the start point of the ST segment is marked as point J, and the end point of the ST segment is marked as point K; — S5

step S6: performing average filtering on an electrocardiogram signal s(t) to obtain an average value sequence, defaulting the average value sequence as a baseline sequence $X_s$, and subtracting the baseline sequence $X_s$ from an original electrocardiogram signal s(t) to obtain a new electrocardiogram signal x(t); — S6

step S7: extracting a baseline TP segment from the start point of the TP segment and the start point of the P wave located from step S1 to step S5, extracting a baseline PR segment from the located end point of the P wave and the located start point of the R wave, extracting a QQ connection line from two located adjacent Q wave peaks, and intercepting an electrocardiogram signal between the point J and the point K as the ST segment; — S7

step S8: calculating a slope of each of the baseline TP segment, the baseline PR segment, and the QQ connection line obtained in step S7, calculating a heart rate of the new electrocardiogram signal x(t), and comprehensively determining a standard baseline for determining the changes of the ST segment; — S8

step S9: with respect to the ST segment obtained in step S7 and the standard baseline obtained in step S8, calculating the difference between the average height of the ST segment and the average height of the baseline, and performing threshold determination according to characteristics of the electrocardiogram signal of different leads to determine whether the ST segment is elevated, depressed or normal. — S9

FIG. 9

```
┌─────────────────────────────────┐
│        First module 201         │
└─────────────────────────────────┘
                 │
┌─────────────────────────────────┐
│       Second module 202         │
└─────────────────────────────────┘
                 │
┌─────────────────────────────────┐
│        Third module 203         │
└─────────────────────────────────┘
                 │
┌─────────────────────────────────┐
│       Fourth module 204         │
└─────────────────────────────────┘
                 │
┌─────────────────────────────────┐
│        Fifth module 205         │
└─────────────────────────────────┘
                 │
┌─────────────────────────────────┐
│        Sixth module 206         │
└─────────────────────────────────┘
                 │
┌─────────────────────────────────┐
│      Seventh module 207         │
└─────────────────────────────────┘
                 │
┌─────────────────────────────────┐
│       Eighth module 208         │
└─────────────────────────────────┘
                 │
┌─────────────────────────────────┐
│        Ninth module 209         │
└─────────────────────────────────┘
```

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011040200 A1